# EUROPEAN PATENT SPECIFICATION

(11) **EP 2 564 207 B1**
(45) Date of publication and mention of the grant of the patent: **12.10.2016**
(21) Application number: 11775191.7
(22) Date of filing: 12.04.2011
(51) Int. Cl.: G01N 33/543, G01N 21/07, B82Y 15/00, G01N 21/64

(54) **CENTRIFUGAL MICRO-FLUIDIC DEVICE AND METHOD FOR IMMUNOASSAY**
MIKROFLUIDISCHE ZENTRIFUGALVORRICHTUNG UND VERFAHREN FÜR EINEN IMMUNTEST
DISPOSITIF MICROFLUIDIQUE CENTRIFUGE ET PROCÉDÉ D'IMMUNO-ANALYSE

(30) Priority: 29.04.2010 KR 20100040370
(43) Date of publication of application: 06.03.2013
(73) Proprietor: Samsung Electronics Co., Ltd, Gyeonggi-do 443-742 (KR)
(72) Inventor: KIM, In Wook, Seongnam-si Gyeonggi-do 463-927 (KR)
(74) Representative: Land, Addick Adrianus Gosling
(86) International application number: PCT/KR2011/002563
(87) International publication number: WO 2011/136485

(56) References cited:
- WO-A1-2009/055587
- WO-A2-2007/106579
- WO-A2-2009/117611
- WO-A2-2010/041230
- US-A1- 2003 166 265
- US-A1- 2004 018 611
- US-A1- 2006 211 055
- US-A1- 2006 263 818
- US-A1- 2008 013 092
- US-A1- 2008 171 400
- US-A1- 2009 181 411
- US-A1- 2009 194 707
- US-A1- 2009 215 088

## Description

### Technical Field

Apparatuses and methods consistent with embodiments relate generally to a centrifugal micro-fluidic device and an immunoassay method using the same and, more particularly, to a micro-fluidic device for detection of analytes in a fluid sample using fluorescent nanoparticles, as well as an immunoassay method using the same.

### Background Art

A micro-fluidic device refers to a device used for conducting biological or chemical reactions using a small amount of fluid.

In general, a micro-fluidic structure of a micro-fluidic device has at least one independent function and includes a chamber containing a fluid therein, a channel through which the fluid flows and a valve for controlling fluid flow. The micro-fluidic structure may be fabricated by combining these components in different ways. In particular, a device referred to as a "lab-on-a-chip" includes a micro-fluidic structure mounted on a substrate in a chip-like platform. With the lab-on-a-chip, some experiments involving biological or chemical reaction can be conducted on a small chip in order to execute several experimental processes and/or operations on the structure. In order to move a fluid within the micro-fluidic structure, a driving pressure is generally required. The driving pressure may be a capillary pressure or pressure generated using an additional pump. In recent years, a disc-type micro-fluidic device referred to as a "Lab CD" (compact disc) or "lab-on-a-disc," has been proposed. The lab-on-a-disc includes a micro-fluidic structure mounted on a disc-type rotational platform which uses centrifugal force to move a fluid in the micro-fluidic structure in order to execute a series of tasks. Efforts are ongoing to develop a variety of disc-type micro-fluidic devices capable of rapidly and precisely conducting desired operations in disc-type platforms using centrifugal force.

However, for immunoassays conducted in general clinical laboratories using conventional disc-type micro-fluidic devices, experimental procedures are typically complex and require a comparatively long time for testing.

US2006/0211055 discloses a microfluidic device which can be incorporated in a rotor. In the related method using the microfluidic device a ternary complex comprising a capture moiety, an analyte and a labeling moiety is formed in a binding channel, the complex is released through a separation channel and detected.

### Disclosure of Invention

The present invention is in its broadest sense defined by the appended claims.

Exemplary embodiments provide a micro-fluidic device for detection of analytes in a fluid sample, such as a liquid specimen. using fluorescent nanoparticles, as well as an immunoassay method using the same.

According to the invention, there is provided a centrifugal micro-fluidic device comprising at least one micro-fluidic structure, the micro-fluidic structure comprising: a sample chamber having a cross-section diameter increasing from a sample introduction inlet toward an outlet for receiving a fluid sample; a separation chamber connected to the sample chamber for centrifugation of the fluid sample for separation of a supernatant containing an analyte from the fluid sample; a first reaction chamber which is connected with the separation chamber and contains at least one labeling conjugate comprising a label and a binder, wherein the hinder specifically binds to the analyte in the supernatant; a second reaction chamber which is connected with the first reaction chamber and contains a capture binder selected from a group comprising: antibody, antigen, receptor, ligand, oligonucleotide, hapten or aptamer; a buffer chamber which is connected with the second reaction chamber and contains an elution buffer, wherein the elution buffer dissociates an immune complex into the labeling conjugate, the analyte and the capture binder; a waste chamber connected to and positioned downstream of the second reaction chamber for receiving impurities including an excess of a first immune complex as well as the analyte; a washer chamber connected to and upstream of the second reaction chamber containing a washing solution for washing away unreacted labeling conjugate and reaction residues; a detection chamber which is connected with the second reaction chamber and receives the at least one dissociated labeling conjugate; a plurality of channels through which the first reaction chamber, second reaction chamber, buffer chamber and detection chamber are interconnected; and at least one valve which is positioned in at least one of the plurality of channels, and opens and closes the channel.

The labeling conjugate may include at least one label selected from a group including: lanthanide (III) chelates or nanoparticles containing lanthanide (III) chelates; colored polymeric nanoparticles; fluorescent materials or nanoparticles containing the same; phosphorescent materials or nanoparticles containing the same; dye-containing liposomes; enzymes (HRP, ALP, etc.); super para-magnetic materials or nanoparticles containing the same; metal nanoparticles; carbon nanoparticles, etc.

The labeling conjugate may be in a dried solid state.

The labeling conjugate may include a label causing expression of optical signals of the analyte in the fluid sample, and wherein the label is combined with the analyte.

The labeling conjugate may contain at least one of various labeling conjugates having individual label substances.

The labeling conjugate includes a binder and a label, wherein the binder is selected from a group including: an antibody, antigen, receptor, ligand, oligonucleotide, hapten or aptamer.

The capture binder may be bonded to a reaction site of the analyte different from another reaction site where the labeling conjugate reacts with the analyte.

The capture binder is selected from a group including: an antibody, antigen, receptor, ligand, oligonucleotide, hapten or aptamer.

The second reaction chamber may have a detection region in which the capture binder is fixed thereto.

The micro-fluidic device has a separation chamber connected with the first reaction chamber, wherein the separation chamber separates a supernatant containing an analyte from the fluid sample.

A detection unit may be positioned outside the micro-fluidic structure, wherein the detection unit includes: a light emission unit emitting light to the detection chamber of the micro-fluidic structure; a light receiving unit receiving the light emitted from the light emitting unit which passed through the detection chamber; and an analysis unit analyzing at least one optical feature of the light received by the light receiving unit and calculating a concentration of at least one analyte in the fluid sample.

According to an aspect of another exemplary embodiment, there is provided an immunoassay method using the above centrifugal micro-fluidic device, the method including the steps of: injecting a fluid sample into the micro-fluidic device, centrifuging the fluid sample to obtain a supernatant, and transferring the supernatant into a first reaction chamber; combining an analyte contained in the supernatant with a labeling conjugate contained in the first reaction chamber, in order to form a first immune complex, wherein the binder specifically binds to the analyte in the supernatant ; combining the first immune complex with a capture binder selected from a group comprising: antibody, antigen, receptor, ligand, oligonucleotide, hapten or aptamer contained in a second reaction chamber to form a second immune complex ; transferring un-reacted labeling conjugate as well as reaction residues to a waste chamber together with a washing solution fed from a washer chamber; disassociating the labeling conjugate from the second immune complex in the second reaction chamber using an elution buffer received from a buffer chamber of the micro-fluidic device ; transferring the dissociated labeling conjugate into a detection of the micro-fluidic device; and measuring and analyzing optical features of the label of the dissociated labeling conjugate in the detection chamber.

The labeling conjugate may include at least one label selected from a group including: lanthanide (III) chelates or nanoparticles containing lanthanide (III) chelates; colored polymeric nanoparticles; fluorescent materials or nanoparticles containing the same; phosphorescent materials or nanoparticles containing the same; dye-containing liposomes; enzymes (HRP, ALP, etc.); super para-magnetic materials or nanoparticles containing the same; metal nanoparticles; carbon nanoparticles, etc.

The labeling conjugate may be in a dried solid state.

The labeling conjugate may include a label causing expression of optical signals of the analyte in the fluid sample, and wherein the label is specifically combined with the analyte.

The labeling conjugate may be at least one of various labeling conjugates having individual label substances.

The labeling conjugate includes a binder and a label, wherein the binder is selected from a group including: an antibody, antigen, receptor, ligand, oligonucleotide, hapten or aptamer.

The capture binder may be bonded to a reaction site of the analyte different from another reaction site where the labeling conjugate reacts with the analyte.

The capture binder is selected from a group including: an antibody, antigen, receptor, ligand, oligonucleotide, hapten or aptamer.

The second reaction chamber may further include a detection region in which the capture binder is fixed thereto.

The fluid sample, supernatant or buffer is transferred by a driving pressure, such as centrifugal force generated by rotation of the micro-fluidic structure.

Determination of fluorescence of the labeling conjugate may be performed by time-resolved fluorescence measurement that enables fine division (that is, resolution) of time, and which measures fluorescence of light received by a light receiving unit during a resolved time.

Fluorescence of the light received by the light receiving unit is measured after a pre-determined time delay.

### Advantageous Effects of Invention

The micro-fluidic device and the immunoassay method according to exemplary embodiments, have characteristics in that: i) multiple labeling conjugates containing individual label substances may be at the same time, in turn enabling simultaneous detection of multiple analytes; ii) the labeling conjugate is used in a dried solid state and can be stored at room temperature instead of cold storage, thus improving applicability to actual clinical environments; iii) the number of washing and the numbers of buffers and valves used for opening and closing the channels are decreased, thus being simpler than conventional test procedures; iv) lanthanide (III) chelate-containing nanoparticles with considerable difference between excitation wavelength and emission wavelength are used as the label substance, and fluorescence of the emitted light is determined by time-resolved fluorescence measurement, thereby remarkably enhancing accuracy and sensitivity in fluorescence measurement of the label substance. Therefore, the micro-fluidic device and the immunoassay method using the same according to exemplary embodiments may be used as a rapid on-site inspection technology.

### Brief Description of Drawings

The above and/or other aspects will become apparent and more readily appreciated from the following description of the exemplary embodiments, taken in conjunction with the accompanying drawings of which:
FIG. 1 is a schematic view illustrating the construction of a micro-fluidic structure, according to an exemplary embodiment;
FIGS. 2 to 5 are conceptual views illustrating a process of combining an analyte with a labeling conjugate and a capture binder, according to an exemplary embodiment;
FIG. 6 is a flowchart illustrating an immunoassay method, according to an exemplary embodiment;
FIG. 7 is a block diagram illustrating a detection unit, according to an exemplary embodiment;
FIG. 8 is a graph depicting optical features of a label substance, according to an exemplary embodiment;
FIG. 9 is a graph illustrating a time-resolved fluorescence measurement method, according to an exemplary embodiment; and
FIG. 10 is a graph depicting a standard curve, according to an exemplary embodiment.

### Best Mode for Carrying out the Invention

Hereinafter, the centrifugal micro-fluidic device and the immunoassay method using the same according to exemplary embodiments will be described with reference to the accompanying drawings.

The same numerical symbols in the drawings refer to substantially the same constitutional elements. Separate structures such as a chamber, a channel, and the like are simply illustrated and dimensional ratios of the same may be different from real scales thereof, instead being enlarged or reduced. Expressions "micro-fluidic device," "micro-particle," etc., "micro" are not limitedly construed as a size unit but used in contrast with "macro."

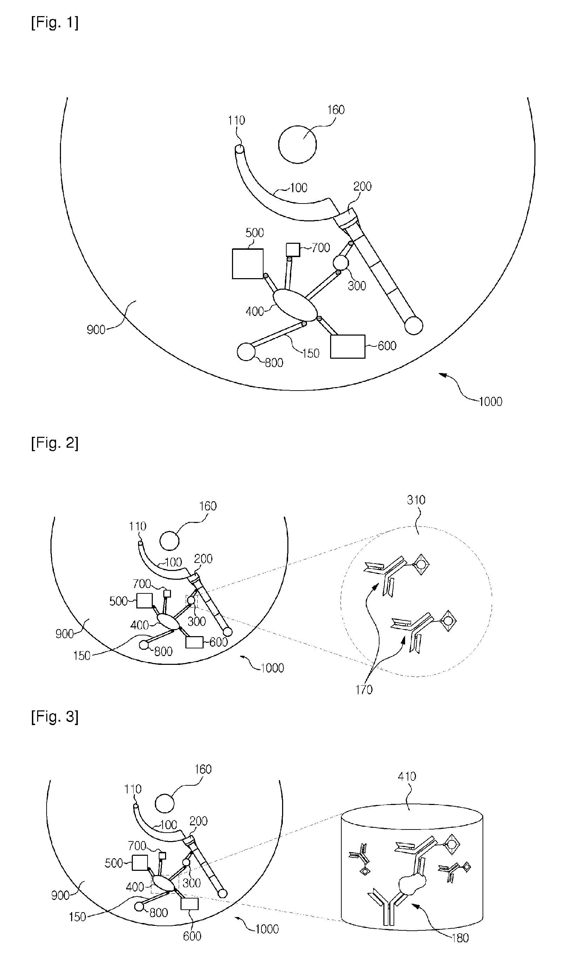

FIG. 1 is a schematic view illustrating the construction of a micro-fluidic structure, according to an exemplary embodiment.

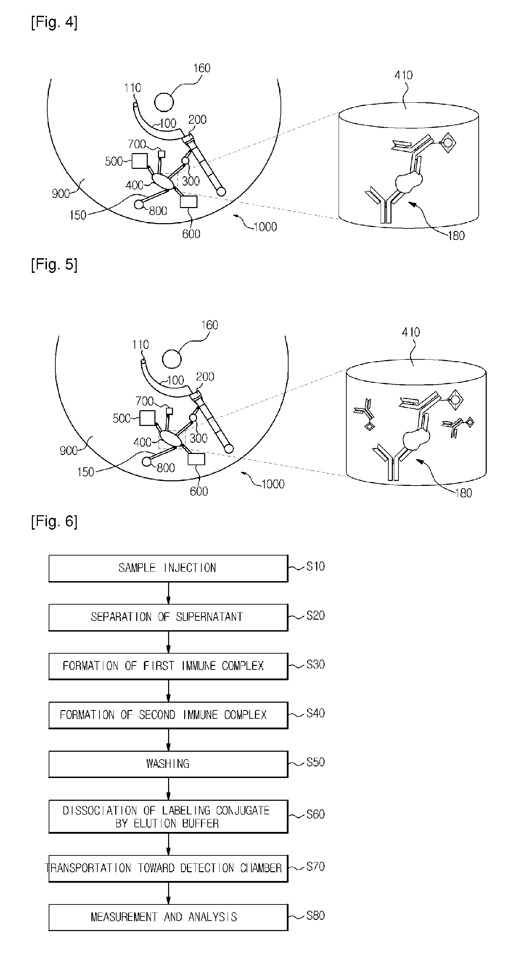

According to the exemplary embodiment in FIG. 1, there is provided a centrifugal micro-fluidic device including: a rotational body 900 and at least one micro-fluidic structure 1000. The at least one micro-fluidic structure 1000 further includes a sample chamber 100 for receiving a fluid sample; a separation chamber 200 for centrifugation of the fluid sample to separate a supernatant containing an analyte; a first reaction chamber 300 containing at least one labeling conjugate; a second reaction chamber 400 containing a capture binder; a buffer chamber 500 containing an elution buffer; a waste chamber 600 for receiving impurities including an excess of a first immune complex as well as the analyte; a washer chamber 700 containing a washing solution; and a detection chamber 800 for ultimately receiving the labeling conjugate. The micro-fluidic structure also includes a plurality of channels 120 through which the multiple chambers are interconnected; at least one valve (not shown) for opening and closing the plurality of channels; and at least one detection unit 10 (see FIG. 4).

Referring to FIG. 1, the rotational body 900 used in the exemplary embodiment may include a circular disc-type platform. However, a shape of the platform is not particularly limited to such circular disc form. That is, the rotational body may be a complete circular shape capable of rotating by itself or a rotatable sector shape placed on a rotational frame. The platform may be formed using acryl or other plastic materials, each of which is easily formed and has a biologically inactive surface. However, a raw material for fabrication of the rotational body is not particularly limited and may include any materials with chemical or biological stability, optical transparency and/or mechanical workability.

The platform may be fabricated using at least one material selected from a variety of materials, such as plastic, polymethylmethacrylate (PMMA), glass, mica, silica, or a silica wafer material. The plastic material may be chosen in view of its economical merits or simple workability. Commonly available plastic materials include polypropylene, polyacrylate, polyvinylalcohol, polyethylene, PMMA, polycarbonate, etc.

One or more micro-fluidic structures may be provided on the platform. For instance, after partitioning the platform into several sections, individual micro-fluidic structures may be placed independently of one another on the sections, respectively.

Also, the platform may include multiple layers of plates (not shown). If a relief structure corresponding to a chamber or a channel is formed on a side at which two plates face each other and two or more relief structures are combined, an empty space and/or channel may be provided inside the platform. Such combination of plates may be achieved using an adhesive, two-sided adhesive tape, ultrasonic welding, etc.

The term "micro-fluidic structure" as used herein refers to a general structure which consists of a plurality of chambers, channels and valves designed to induce a fluid flow, as opposed to a particular structural substance. Therefore, the "micro-fluidic structure" may form a specific unit with different functions or performances according to features provided by the arrangement of chambers, channels and/or valves, and/or kinds of materials contained in the structure.

Accordingly, the micro-fluidic device has a wide variety of applications, such as detection of various chemical compounds, environmentally harmful substances, blood analysis, urine testing, antigen-antibody response-based immunoassay, novel drug candidate searches based on ligand-receptor binding, DNA/RNA analysis, and so forth. Further, the micro-fluidic device may simultaneously detect and analyze at least two analytes.

A fluid sample such as a blood sample, a supernatant of the fluid sample, a buffer solution, etc. may be transported into separate chambers using centrifugal force generated by rotation of the rotational body 900 as a driving pressure.

When the fluid sample is fed into the micro-fluidic structure 1000 through a sample introduction inlet 110, the fluid sample is substantially received by the sample chamber 100.

The sample chamber 100 may offer an empty space in which a fluid sample such as a blood sample is contained. The sample chamber 100 has the sample introduction inlet 110 through which the sample is provided to the micro-fluidic structure 1000, and a sample receiving part (not shown). The sample receiving part also has an outlet connected to the separation chamber 200. Although not illustrated in the drawings, the outlet may have a desired shape designed to create capillary pressure that prevents the fluid sample from moving toward the separation chamber 200 when centrifugal force is not being applied. Alternatively, the outlet may have a valve mounted thereon in order to control a flow of the fluid sample. Furthermore, the sample chamber 100 is fabricated to have a cross-section diameter increasing from the sample introduction inlet 100 toward the outlet, enabling the sample contained in the sample receiving part to easily flow toward the separation chamber 200 by centrifugal force. In order to facilitate flow of the sample into the sample receiving part by injection pressure of the sample through the sample introduction inlet 110 and, an alternative structure to generate capillary pressure may be placed between the sample introduction inlet 110 and the sample receiving part. The alternative structure may be a capillary valve-type structure which passes the sample through the same only when a desired pressure is applied, and it may also serve to block reverse flow of the sample from the sample receiving part toward the sample introduction inlet 110.

The fluid sample is delivered toward the separation chamber 200 using centrifugal force generated by rotation of the rotational body 900 as a driving pressure. The separation chamber 200 is located radially outward from the sample chamber 100; that is, further from a center 160 of the rotational body 900 than the sample chamber 100. The sample separation chamber 200 may enable centrifugation of the fluid sample into a supernatant (serum, plasma, etc.) and a precipitate (blood cells). The fluid sample in the separation chamber 200 is separated using centrifugal force into a supernatant containing analytes and a precipitate containing other materials.

The separation chamber 200 for centrifugation of the fluid sample may be configured in different forms. Most particularly, the separation chamber 200 may include a supernatant collector (not shown) and a precipitate collector (not shown) as a space being formed at an end of the supernatant collector to collect a precipitate with relatively high specific gravity. The supernatant collector has a channel for dispensing the centrifuged supernatant into the first reaction chamber 300. A valve may control flow of the sample through the channel. Such a valve may be any type of valve selected from different types of micro-fluidic valves. For example, the valve may include a so-called "normally closed valve" wherein a channel in which the valve is located is closed to prevent a fluid from flowing unless the valve opens by external power.

A supernatant metering chamber (not shown) may be placed on the separation chamber 200 to measure an amount of the supernatant. The supernatant metering chamber may be designed with a volume sufficient to carry an amount of the supernatant required for testing.

The supernatant containing the analyte may be transported from the separation chamber 200 to the first reaction chamber 300 through a channel 150 using centrifugal force generated by rotation of the rotational body 900 as a driving pressure. The first reaction chamber 300 contains a labeling conjugate, as illustrated by the conceptual exploded view 310 of the first reaction chamber 300 in FIG. 2 and described further below.

The first reaction chamber 300 is a structure for detecting specific protein, glucose, cholesterol, uric acid, creatinine, alcohol, etc., contained in the supernatant. The detection may be accomplished by antigen-antibody response, ligand-receptor binding, and so forth.

The labeling conjugate consists of a label and a binder. Labels of the labeling conjugate may include, for example, latex beads; metal colloids such as gold colloids, silver colloids, etc.; enzymes (enzyme, HRP, ALP, etc.); colored materials; fluorescent materials or nanoparticles containing the same; phosphorescent materials or nanoparticles containing the same; luminous materials; light emitting materials; dye-containing liposomes; metal nanoparticles; carbon nanoparticles; colored polymeric nanoparticles; super para-magnetic materials or nanoparticles containing the same; lanthanide (III) chelates or nanoparticles containing the same; radioactive isotopes; etc. However, the labels are not particularly limited thereto. According to an exemplary embodiment, lanthanide (III) chelate-containing nanoparticles are used as a label substance. Non-limiting examples of lanthanide (III) chelates may include europium (Eu), samarium (Sm), dysprosium (Dy), terbium (Tb), etc. The binder may include an antibody, antigen, ligand, receptor, oligonucleotide, hapten or aptamer; each of which can be specifically bonded to the analyte in the supernatant.

According to an exemplary embodiment, the first reaction chamber 300 of the micro-fluidic device may contain a single labeling conjugate containing one kind of label substance in order to detect one analyte; however, the first reaction chamber 300 may also contain several labeling conjugates containing different label substances in order to simultaneously detect plural analytes. For instance, in order to detect four types of distinct analytes at the same time, four types of labeling conjugates containing four different lanthanide (III) chelates as separate label substances, such as Eu, Sm, Dy and Tb, may be contained together in the first reaction chamber 300.

The above configuration may have advantages in that a single micro-fluidic structure 1000 may be used to detect multiple analytes. Multiple analytes may also be detected at the same time by using only one micro-fluidic device having several micro-fluidic structures built therein. This configuration is advantageous when compared to conventional micro-fluidic devices required for detection of multiple analytes.

As described above, the micro-fluidic device according to the exemplary embodiment may simultaneously detect plural analytes which provides for rapid inspection and economical merits, and is therefore applicable to situations where rapid on-site inspection is desired.

The labeling conjugate may be present in a liquid or a dried solid phase. Since a liquid phase of the labeling conjugate requires cold transportation and storage in order to retain stability thereof, these requirements make the liquid labeling conjugate less available in practical clinical environments. Therefore, the labeling conjugate is preferably present in a dried solid phase.

When the labeling conjugate in a solid phase is present in the reaction chamber 300, the labeling conjugate may be temporarily fixed to an inner wall of the reaction chamber 300 or a porous support therein (not shown). The labeling conjugate fixed to the reaction chamber 300 is lysed by penetration of the supernatant, after which the lysed conjugate is combined with an analyte contained in the supernatant to form a first immune complex 170, as illustrated in FIG. 2. The first immune complex 170 becomes a movable product. In this case, in order to facilitate combination of the labeling conjugate and the analyte in the supernatant, the rotational body 900 may be preferably shaken several times to the right and left.

Centrifugal force generated by rotation of the rotational body 900 is used as a driving pressure to transport the supernatant containing the first immune complex 170 toward the second reaction chamber 400 through a channel 150.

The second reaction chamber 400 is a structure for detecting some materials contained in the supernatant including, for example, specific protein, glucose, cholesterol, uric acid, creatinine, alcohol, etc. The materials may be detected using antigen-antibody response or ligand-receptor binding, and so forth.

The second reaction chamber 400 may contain the capture binder fixed in a detection region, wherein the capture binder is specifically combined with the analyte contained in the supernatant fed from the first reaction chamber 300.

The first immune complex in the transported supernatant from the first reaction chamber 300 is combined with the capture binder placed in the detection region to form a second immune complex 180, as illustrated by the conceptual exploded view 410 of the second reaction chamber 400 in FIG. 3. In this case, in order to facilitate combination of the first immune complex 170 and the capture binder, the rotational body 900 may be shaken several times to the right and left. Un-reacted labeling conjugate in the reactive solution as well as reaction residues are transferred to the waste chamber 600 together with a washing solution fed from the washer chamber 700, while the second immune complex 180 remains in the second reaction chamber 400, as illustrated by the conceptual exploded view 410 of the second reaction chamber in FIG. 4.

When the second immune complex 180 remains after the washing process, an elution buffer is delivered from the buffer chamber 500 located above the second reaction chamber 400 to the second reaction chamber 400 by centrifugal force as a driving pressure. The delivered elution buffer dissociates antigen-antibody bonds or ligand-receptor bonds of the second immune complex fixed to the detection region in the second reaction chamber 400, in turn enabling dissociation (or degradation) of the labeling conjugate and the analytes from the fixed capture binder. In other words, both the labeling conjugate and the capture binder are dissociated with reference to the analytes, as illustrated by the conceptual exploded view 410 of the second reaction chamber in FIG. 5.

Conventional technologies generally i) directly determine optical features of a label substance without alternative dissociation of the labeling conjugate from the immune complex; or ii) measure fluorescence of the label substance after dissociating the label substance.

However, the exemplary embodiments described herein provide dissociation of the label substance and the capture binder from the second immune complex using the elution buffer from the buffer chamber 500, with reference to the analyte in the second immune complex (see FIG. 5).

Such procedures proposed by the exemplary embodiments herein may improve sensitivity and accuracy in measurement of label substances. Other methods for dissociation of a label substance generally include attachment of at least one chemical material corresponding to the label substance to a labeling conjugate and dissociation thereof. These procedures may experience technical difficulties and may not improve accuracy and sensitivity in measurement of the label substance as compared to measurement of a group of various label substances.

The process for direct determination of optical features of the label substance contained in the immune complex without alternative separation of the labeling conjugate from the immune complex duly entails decreased accuracy and sensitivity in measurement of optical features, compared to a conventional technique for measurement of optical features of the label substance after removal of the labeling conjugate. For instance, since a mechanical structure (i.e., beads) to which the immune complex is attached can reflect the incident light to determine optical features of the label substance, the structure may adversely influence measurement of optical features of the label substance.

Accordingly, a method for determining optical features of a label substance in a labeling conjugate after dissociation of the labeling conjugate and capture binder from the analyte, as described in an exemplary embodiment, may be easily embodied compared to conventional processes. The label substance may include lanthanide (III) chelate (i.e., Eu) containing nanoparticles as a label substance. The method may attain improvements in accuracy and sensitivity in measurement.

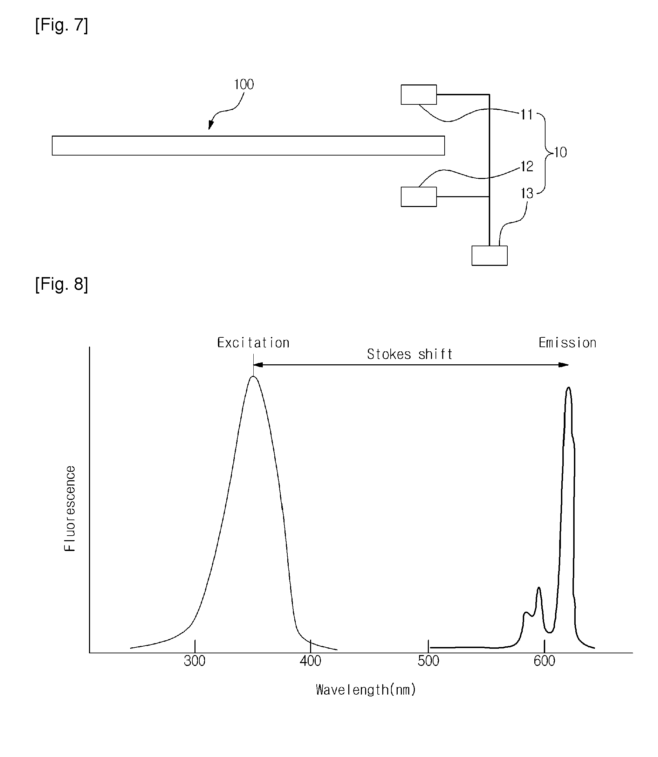

The supernatant containing the labeling conjugate dissociated from the second immune complex is transported into the detection chamber 800 below the second reaction chamber 400 using centrifugal force generated by rotation of the rotational body 900 as a driving pressure. For the labeling conjugate fed into the detection chamber 800, optical features are determined by the detection unit 10 (see FIG. 7) placed outside the micro-fluidic structure 1000. This process will be more apparent from the following description for a method for immunoassay using the micro-fluidic device according to an exemplary embodiment.

FIG. 6 is a flowchart illustrating an immunoassay method using the micro-fluidic device according to an exemplary embodiment. In this exemplary embodiment, a process for blood analysis is exemplified.

For example, whole blood is injected into a sample chamber 100 (S10), and a micro-fluidic structure is rotated to deliver the whole blood toward a separation chamber 200. The whole blood is transported from the sample chamber 100 to the separation chamber 200 using centrifugal force generated by rotation of a rotational body 900 as a driving pressure.

The whole blood fed into the separation chamber 200 is separated by high speed revolution (S20) into a supernatant containing a serum or plasma and precipitate containing blood cells. In this regard, blood cells thicker (or heavier) than the serum or plasma may precipitate and move to a precipitate collector, while the supernatant, which is lighter than the blood cells, remains in the supernatant collector.

When opening a valve, the separated supernatant is delivered to a first reaction chamber 300 through a channel by a driving pressure, that is, centrifugal force generated by rotation of the rotational body 900. The labeling conjugate is in a dried solid state, is contained in the first reaction chamber 300 and is re-dissolved by inflow of the supernatant and specifically combined with the analyte contained in the supernatant to form a first immune complex (S30). During this process, in order to facilitate combination of the analyte and the labeling conjugate, the rotational body 900 may be shaken several times to the right and left.

When opening a valve, the supernatant containing the first immune complex is delivered to a second reaction chamber 400 using centrifugal force generated by rotation of the rotational body 900 as a driving pressure.. The capture binder is contained within the second reaction chamber 400 and may be fixed to the detection region of the second reaction chamber 400. The capture binder is specifically combined with the analyte to form a second immune complex (S40). During this process, in order to facilitate combination of the first immune complex and the capture binder, the rotational body 900 may be shaken several times to the right and left.

In order to transfer an un-reacted labeling conjugate and reaction residues into a waste chamber 600 positioned downstream of the second reaction chamber 400, a valve connected to a washer chamber 700 upstream of the second reaction chamber 400 is opened, and a washing solution is fed into the second reaction chamber 400 through a channel using centrifugal force generated by rotation of the rotational body 900 as a driving pressure (S50).

After the washing process, another valve connected to a buffer chamber 500 located upstream of the second reaction chamber 400 is opened, and an elution buffer of the buffer chamber is fed into the second reaction chamber 400 through a channel, using centrifugal force generated by rotation of the rotational body 900 as a driving pressure. The elution buffer allows the dissociation of the labeling conjugate from the second immune complex, with reference to analytes contained in the second immune complex (S60).

After dissociation of the labeling conjugate by the elution buffer, a valve is opened and the supernatant containing the labeling conjugate is transported into the detection chamber 800 through a channel using centrifugal force generated by rotation of the rotational body 900 as a driving pressure (S70).

After the supernatant containing the labeling conjugate is fed into the detection chamber 800, the detection unit 10 placed outside the micro-fluidic structure 1000 starts measurement and analysis of optical features of the label substance of the labeling conjugate (S80).

Such measurement and analysis of optical features of the label substance may be performed by irradiating the label substance with light at a desired wavelength range, measuring fluorescence of the light emitted from the label substance at a specific wavelength range, analyzing the measured value, and calculating a concentration of an analyte based on the analyzed result.

Measurement and analysis of optical features of the label substance may be conducted using the detection unit 10 placed outside the micro-fluidic structure 1000, as illustrated in FIG. 7.

The detection unit 10 is substantially placed outside the micro-fluidic structure 1000, and multiple units may be present. Such a detection unit may include a light emission unit 11 to radiate light to the detection chamber 800 of the micro-fluidic structure 1000, a light receiving unit 12 to receive light emitted from the detection chamber 800 which absorbs the light emitted from the light receiving part 12, and an analysis part to analyze optical features of the light received by the light receiving part 12 and calculate a concentration of an analyte based on the analyzed result.

The light emission part 11 may be a light source flashing at a specific frequency including, for example, a semiconductor light emitting device such as an LED or a laser diode (LD), a gas discharge lamp such as a halogen lamp or a xenon lamp, etc.

The light receiving part 12 generates electrical signals according to an intensity of the light emitted from the detection chamber 800 and may include, for example, a depletion layer photodiode, avalanche photodiode (APD), photomultiplier tube (PMT), etc.

In the present exemplary embodiment, the light emission part 11 is located above the micro-fluidic structure 1000 while the light receiving part 12 is positioned below the micro-fluidic structure 1000, however, the positions of these parts may be switched. Also, a light path may be adjusted using a reflecting mirror or a light guide member.

The analysis part 13 determines fluorescence of the light received by the light receiving part and calculates a concentration of an analyte using a pre-determined standard curve identifying the determined fluorescence of the received light,.

In an exemplary embodiment, lanthanide (III) chelate containing nanoparticles are used as a label substance. The following description will be given of using Eu as an example of the lanthanide (III) chelate. Eu exhibits a specific optical feature of providing a considerable difference between excitation wavelength and emission wavelength, as illustrated in the graph in FIG. 8.

Such an optical feature of Eu that is excited by light at a specific wavelength and discharges light at another wavelength considerably different from the excitation wavelength may remarkably improve accuracy in measurement of fluorescence of the emitting light.

Other than Eu as the label substance, foreign materials contained in the detection chamber 800 also absorb and emit the incident light. Therefore, the optical feature of Eu, that is, emission of light at a wavelength considerably different from a wavelength of the incident light may prevent interference of the foreign materials to the emitting light in measurement of the light emitted from the label substance. As a result, fluorescence of the light emitted from the label substance may be more precisely determined.

Based on such features of Eu, influences of the light emitted by foreign materials can be eliminated and fluorescence of the emitting light can be determined by time-resolved fluorescence measurement, in turn enhancing accuracy in measurement of fluorescence.

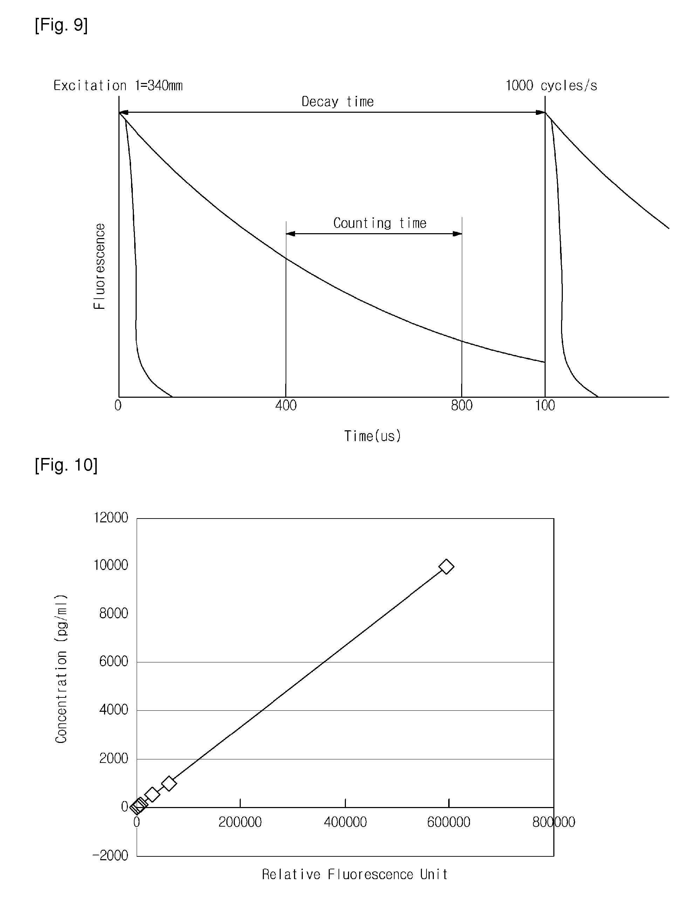

Herein, 'time-resolved fluorescence measurement' refers to a method of finely resolving a constant period of time for measurement and measuring fluorescence per resolved time. For instance, as to measurement of fluorescence of the light emitted for 1 second, the fluorescence may be measured at every ms. Therefore, the same cycle may be repeated 1000 times per second (see FIG. 9).

Referring to FIG. 9, it can be seen that fluorescence measurement is executed in the range of 400 microseconds (µs) to 800µs. That is, fluorescence measurement is performed after a constant delay time, instead of direct measurement. Indeed, since the detection chamber 800 containing the label substance and/or foreign materials possibly fed into the detection chamber 800, other than the label substance, may also absorb the incident light and emit the absorbed light, the foregoing delay time is required to eliminate influence of the foreign materials and/or the detection chamber.

By fluorescence measurement, influence of the light emitted by some materials other than the label substance may be eliminated, thereby enhancing accuracy and sensitivity in measurement of fluorescence.

Exemplary embodiments propose a method of noticeably improving accuracy and sensitivity in measurement of fluorescence of a label substance by: i) using lanthanide (III) chelate containing nanoparticles (i.e., Eu) as the label substance; and ii) applying time-resolved fluorescence measurement. Briefly, an exemplary embodiment describes an immunoassay method of analytes with superior accuracy and sensitivity by combination of the foregoing procedures, compared to conventional methods for measurement of analytes in fluid specimens.

Based on fluorescence of the label substance determined according to the foregoing method, a concentration of an analyte may be calculated using a pre-determined standard curve that shows a concentration of the analyte relative to fluorescence of the label substance, as illustrated in FIG. 10.

## Claims

1. A centrifugal micro-fluidic device comprising at least one micro-fluidic structure, the micro-fluidic structure comprising:
a sample chamber having a cross-section diameter increasing from a sample introduction inlet toward an outlet for receiving a fluid sample;
a separation chamber connected to the sample chamber for centrifugation of the fluid sample for separation of a supernatant containing an analyte from the fluid sample;
a first reaction chamber which is connected with the separation chamber and contains at least one labeling conjugate comprising a label and a binder, wherein the binder specifically binds to the analyte in the supernatant;
a second reaction chamber which is connected with the first reaction chamber and contains a capture binder selected from a group comprising: antibody, antigen, receptor, ligand, oligonucleotide, hapten or aptamer;
a buffer chamber which is connected with the second reaction chamber and contains an elution buffer, wherein the elution buffer dissociates an immune complex into the at least one labeling conjugate, the analyte and the capture binder;
a waste chamber connected to and positioned downstream of the second reaction chamber for receiving impurities including an excess of a first immune complex as well as the analyte;
a washer chamber connected to and upstream of the second reaction chamber containing a washing solution for washing away unreacted labeling conjugate and reaction residues;
a detection chamber which is connected with the second reaction chamber for receiving the at least one dissociated labeling conjugate;
a plurality of channels through which the first reaction chamber, second reaction chamber, buffer chamber and detection chamber are interconnected; and
at least one valve which is positioned in at least one of the plurality of channels, and opens and closes the channel.

2. The micro-fluidic device according to claim 1, wherein the labeling conjugate comprises at least one label selected from a group comprising: lanthanide (III) chelates or nanoparticles containing the same; colored polymeric nanoparticles; fluorescent materials or nanoparticles containing the same; phosphorescent materials or nanoparticles containing the same; dye-containing liposomes; enzymes; super para-magnetic materials or nanoparticles containing the same; metal nanoparticles; and carbon nanoparticles.

3. The micro-fluidic device according to claim 1 or 2, wherein the labeling conjugate comprises a label causing expression of optical signals of the analyte in the fluid sample, and the label is combined with the analyte.

4. The micro-fluidic device according to any of claims 1-3, wherein the labeling conjugate is at least one of various labeling conjugates containing individual label substances.

5. The micro-fluidic device according to any of claims 1-4, wherein the binder is selected from a group comprising: antibody, antigen, receptor, ligand, oligonucleotide, hapten and aptamer.

6. The micro-fluidic device according to any of claims 1-5, wherein the capture binder is bonded to a reaction site of an analyte in the fluid sample that is different from another reaction site where the labeling conjugate reacts with the analyte.

7. The micro-fluidic device according to any of claims 1-6, wherein the second reaction chamber comprises a detection region in which the capture binder is fixed thereto.

8. The micro-fluidic device according to any of claims 1-7, further comprising a detection unit positioned outside the micro-fluidic structure, the detection unit comprising:
a light emission unit that emits light to the detection chamber of the micro-fluidic structure;
a light receiving unit that receives the light emitted from the light emitting unit which passed through the detection chamber, and
an analysis unit that analyzes at least one optical feature of the light received by the light receiving unit and calculates a concentration of the analyte in the fluid sample.

9. An immunoassay method using a centrifugal micro-fluidic device according to any of claims 1 to 8, the immunoassay method comprising:
injecting a fluid sample into the micro-fluidic device, centrifuging the fluid sample to obtain a supernatant, and transferring the supernatant into a first reaction chamber of the micro-fluidic device;
combining an analyte contained in the supernatant with a labeling conjugate comprising a label and a binder contained in the first reaction chamber to form a first immune complex, wherein the binder specifically binds to the analyte in the supernatant;
combining the first immune complex with a capture binder selected from a group comprising: antibody, antigen, receptor, ligand, oligonucleotide, hapten or aptamer contained in a second reaction chamber to form a second immune complex;
transferring un-reacted labeling conjugate as well as reaction residues to a waste chamber together with a washing solution fed from a washer chamber;
disassociating the labeling conjugate from the second immune complex in the second reaction chamber using an elution buffer received from a buffer chamber of the micro-fluidic device;
transferring the dissociated labeling conjugate into a detection chamber of the micro-fluidic device; and
measuring and analyzing optical features of the label of the dissociated labeling conjugate in the detection chamber.

10. The immunoassay method according to claim 9, wherein the labeling conjugate includes at least one label selected from a group comprising: lanthanide (III) chelates or nanoparticles containing the same; colored polymeric nanoparticles; fluorescent materials or nanoparticles containing the same; phosphorescent materials or nanoparticles containing the same; dye-containing liposomes; enzymes; super para-magnetic materials or nanoparticles containing the same; metal nanoparticles; and carbon nanoparticles.

11. The immunoassay method according to claim 9 or 10, wherein the labeling conjugate is at least one of various labeling conjugates containing individual label substances.

12. The immunoassay method according to claim 9, 10 or 11, wherein the binder is selected from a group comprising: antibody, antigen, receptor, ligand, oligonucleotide, hapten or aptamer.

13. The immunoassay method according to any of claims 9-12, wherein the capture binder is bonded to a reaction site of the analyte that is different from another reaction site where the labeling conjugate reacts with the analyte.

14. The immunoassay method according to any of claims 9-13, wherein the second reaction chamber comprises a detection region in which the capture binder is fixed thereto.

15. The immunoassay method according to any of claims 9-14, further comprising determining fluorescence of the labeling conjugate using time-resolved fluorescent measurement that measures fluorescence of light received by a light receiving unit of the detection unit during a resolved time, wherein fluorescence of the light received by the light receiving unit is measured after a predetermined time delay.

## Patentansprüche

1. Mikrofluidische Zentrifugalvorrichtung umfassend wenigstens eine mihrofluidische Struktur, wobei die mihrofluidische Struktur umfasst:
eine zum Empfang einer Fluidprobe ausgelegte Probenkammer mit einem Querschnittsdurchmesser, der sich von einem Einlass zur Probenzufuhr hin zu einem Auslass vergrößert;
eine mit der Probenkammer verbundene Trennkammer zum Zentrifugieren der Fluidprobe, um einen Überstand, der ein Analyt enthält, von der Fluidprobe zu trennen;
eine erste Reaktionskammer, die mit der Trennkammer verbunden ist und wenigstens ein Markierungskonjugat mit einem Markierungsmittel und einem Bindemittel enthält, wobei das Bindemittel sich spezifisch an das Analyt in dem Überstand bindet;
eine zweite Reaktionskammer, die mit der ersten Reaktionskammer verbunden ist und ein Einfangbindemittel enthält, welches aus einer Gruppe umfassend die folgenden ausgewählt wird: Antikörper, Antigen, Rezeptor, Ligand, Oligonukleotid, Hapten oder Aptamer;
eine Pufferkammer, die mit der zweiten Reaktionskammer verbunden ist und einen Elutionspuffer enthält, wobei der Elutionspuffer einen Immunkomplex in das wenigstens eine Markierungskonjugat, das Analyt und das Einfangbindemittel aufspaltet;
eine mit der zweiten Reaktionskammer verbundene und stromabwärts von dieser angeordnete Abfallkammer zur Aufnahme von Unreinheiten umfassend einen Überschuss eines ersten Immunkomplexes sowie dem Analyt;
eine mit der zweiten Reaktionskammer verbundene und stromaufwärts von dieser angeordnete Waschkammer, die eine Waschlösung zum Abwaschen von unreagiertem Markierungskonjugat und von Reaktionsrückständen enthält;
eine mit der zweiten Reaktionskammer verbundene Nachweiskammer zur Aufnahme des wenigstens einen abgespaltenen Markierungskonjugats;
eine Mehrzahl von Kanälen, durch die die erste Reaktionskammer, die zweite Reaktionskammer, die Pufferkammer und die Nachweiskammer miteinander verbunden sind; und
wenigstens ein Ventil, das in wenigstens einem Kanal aus der Mehrzahl von Kanälen angeordnet ist und den Kanal öffnet und schließt.

2. Mikrofluidische Vorrichtung gemäß Anspruch 1, wobei das Markierungskonjugat wenigstens ein Markierungsmittel enthält, welches aus einer Gruppe umfassend die folgenden ausgewählt wird: Lanthanid (III) Chelate oder Nanopartikel, die diese enthalten; gefärbte polymere Nanopartikel; fluoreszierende Materialien oder Nanopartikel, die diese enthalten; phosphoreszierende Materialien oder Nanopartikel, die diese enthalten; farbstoffhaltige Liposome; Enzyme; super-paramagnetische Materialien oder Nanopartikel, die diese enthalten; Metallnanopartikel; und Kohlenstoff- Nanopartikel.

3. Mikrofluidische Vorrichtung gemäß Anspruch 1 oder 2, wobei das Markierungskonjugat ein Markierungsmittel umfasst, das eine Expression von optischen Signalen des Analyts in der Fluidprobe verursacht, und wobei das Markierungsmittel mit dem Analyt kombiniert ist.

4. Mikrofluidische Vorrichtung gemäß einem der Ansprüche 1 bis 3, wobei es sich bei dem Markierungskonjugat um wenigstens eines von verschiedenen Markierungskonjugaten handelt, das individuelle Markierungssubstanzen enthält.

5. Mikrofluidische Vorrichtung gemäß einem der Ansprüche 1 bis 4, wobei das Bindemittel aus einer Gruppe umfassend die folgenden ausgewählt wird: Antikörper, Antigen, Rezeptor, Ligand, Oligonukleotid, Hapten oder Aptamer.

6. Mikrofluidische Vorrichtung gemäß einem der Ansprüche 1 bis 5, wobei das Einfangbindemittel an einen Reaktionsort eines Analyts in der Fluidprobe gebunden ist, der sich von einem anderen Reaktionsort unterscheidet, an dem das Markierungskonjugat mit dem Analyt reagiert.

7. Mikrofluidische Vorrichtung gemäß einem der Ansprüche 1 bis 6, wobei die zweite Reaktionskammer einen Nachweisbereich, in dem das Einfangbindemittel an diesem befestigt ist, umfasst.

8. Mikrofluidische Vorrichtung gemäß einem der Ansprüche 1 bis 7, weiterhin umfassend eine außerhalb der mikrofluidischen Struktur angeordnete Nachweiseinheit, wobei die Nachweiseinheit umfasst:
eine Lichtabgabeeinheit, die Licht an die Nachweiskammer der mikrofluidischen Struktur abgibt;
eine Lichtempfangseinheit, die das von der Lichtabgabeeinheit abgegebene Licht, das durch die Nachweiskammer passierte, empfängt; und
eine Analyseeinheit, die wenigstens eine optische Eigenschaft des von der Lichtempfangseinheit empfangenen Lichts analysiert und eine Konzentration des Analyts in der Fluidprobe berechnet.

9. Verfahren für einen Immuntest mit Hilfe einer mikrofluidischen Zentrifugalvorrichtung gemäß einem der Ansprüche 1 bis 8, wobei das Verfahren für einen Immuntest umfasst:
Einspritzen einer Fluidprobe in die mihrofluidische Vorrichtung, Zentrifugieren der Fluidprobe, um einen Überstand zu erhalten, und Übertragen des Überstands in eine erste Reaktionskammer der mikrofluidischen Vorrichtung;
Kombinieren eines in dem Überstand enthaltenen Analyts mit einem Markierungskonjugat, das ein Markierungsmittel und ein Bindemittel umfasst und in der ersten Reaktionskammer enthalten ist, um einen ersten Immunkomplex zu bilden, wobei das Bindemittel sich spezifisch an das Analyt in dem Überstand bindet;
Kombinieren des ersten Immunkomplexes mit einem Einfangbindemittel, welches aus einer Gruppe umfassend die folgenden ausgewählt wird: Antikörper, Antigen, Rezeptor, Ligand, Oligonukleotid, Hapten oder Aptamer, das in einer zweiten Reaktionskammer enthalten ist, um einen zweiten Immunkomplex zu bilden;
Übertragen von unreagiertem Markierungskonjugat sowie von Reaktionsrückständen in eine Abfallkammer gemeinsam mit einer Waschlösung, die aus einer Waschkammer zugeführt wird;
Abspalten des Markierungskonjugats von dem zweiten Immunkomplex in der zweiten Reaktionskammer mit Hilfe eines Elutionspuffers, der aus einer Pufferkammer der mikrofluidischen Vorrichtung empfangen wird;
Übertragen des abgespaltenen Markierungskonjugats in eine Nachweiskammer der mikrofluidischen Vorrichtung; und
Messen und Analysieren der optischen Eigenschaften des Markierungsmittels des abgespaltenen Markierungskonjugats in der Nachweiskammer.

10. Verfahren für einen Immuntest gemäß Anspruch 9, wobei das Markierungskonjugat wenigstens ein Markierungsmittel enthält, welches aus einer Gruppe umfassend die folgenden ausgewählt wird: Lanthanid (III) Chelate oder Nanopartikel, die diese enthalten; gefärbte polymere Nanopartikel; fluoreszierende Materialien oder Nanopartikel, die diese enthalten; phosphoreszierende Materialien oder Nanopartikel, die diese enthalten; farbstoffhaltige Liposome; Enzyme; super-paramagnetische Materialien oder Nanopartikel, die diese enthalten; Metallnanopartikel; und Kohlenstoff- Nanopartikel.

11. Verfahren für einen Immuntest gemäß Anspruch 9 oder 10, wobei es sich bei dem Markierungskonjugat um wenigstens eines von verschiedenen Markierungskonjugaten handelt, das individuelle Markierungssubstanzen enthält.

12. Verfahren für einen Immuntest gemäß Anspruch 9, 10 oder 11, wobei das Bindemittel aus einer Gruppe umfassend die folgenden ausgewählt wird: Antikörper, Antigen, Rezeptor, Ligand, Oligonukleotid, Hapten oder Aptamer.

13. Verfahren für einen Immuntest gemäß einem der Ansprüche 9 bis 12, wobei das Einfangbindemittel an einen Reaktionsort des Analyts gebunden ist, der sich von einem anderen Reaktionsort unterscheidet, an dem das Markierungskonjugat mit dem Analyt reagiert.

14. Verfahren für einen Immuntest gemäß einem der Ansprüche 9 bis 13, wobei die zweite Reaktionskammer einen Nachweisbereich, in dem das Einfangbindemittel an diesem befestigt ist, umfasst.

15. Verfahren für einen Immuntest gemäß einem der Ansprüche 9 bis 14, weiterhin umfassend das Bestimmen einer Fluoreszenz des Markierungskonjugats mit Hilfe einer zeitaufgelösten Fluoreszenzmessung, bei der die Fluoreszenz eines von einer Lichtempfangseinheit der Nachweiseinheit empfangenen Lichts während eines aufgelösten Zeitraums gemessen wird, wobei die Fluoreszenz des von der Lichtempfangseinheit empfangenen Lichts nach einer vorbestimmten Zeitverzögerung gemessen wird.

## Revendications

1. Dispositif microfluidique centrifuge comprenant au moins une structure microfluidique, la structure microfluidique comprenant:
une chambre à échantillon destinée à recevoir un échantillon fluide et présentant un diamètre de section transversale croissant entre une entrée d'introduction d'échantillon et une sortie;
une chambre de séparation reliée à la chambre à échantillon, destinée à la centrifugation de l'échantillon fluide afin de séparer de l'échantillon fluide un surnageant contenant un analyte;
une première chambre de réaction qui est reliée à la chambre de séparation et contient au moins un conjugué de marquage comprenant un marqueur et un liant, le liant se liant spécifiquement à l'analyte du surnageant;
une seconde chambre de réaction qui est reliée à la première chambre de réaction et contient un liant de capture choisi dans le groupe comprenant: anticorps, antigène, récepteur, ligand, oligonucléotide, haptène ou aptamère;
une chambre de tampon qui est reliée à la seconde chambre de réaction et contient un tampon d'élution, le tampon d'élution permettant la dissociation d'un complexe immun en l'au moins un conjugué de marquage, l'analyte et le liant de capture;
une chambre de décharge reliée à la seconde chambre de réaction, en aval de celle-ci, et destinée à recevoir des impuretés comportant un excédent d'un premier complexe immun ainsi que l'analyte;
une chambre de lavage reliée à la seconde chambre de réaction, en amont de celle-ci, et contenant une solution de lavage destinée à l'élimination du conjugué de marquage n'ayant pas réagi et des résidus réactionnels;
une chambre de détection qui est reliée à la seconde chambre de réaction pour recevoir l'au moins un conjugué de marquage dissocié;
une pluralité de canaux grâce auxquels la première chambre de réaction, la seconde chambre de réaction, la chambre de tampon et la chambre de détection sont reliées entre elles; et au moins une vanne qui est située dans au moins un des canaux de la pluralité de canaux, et qui ouvre et ferme ce canal.

2. Dispositif microfluidique selon la revendication 1, dans lequel le conjugué de marquage comprend au moins un marqueur choisi dans le groupe comprenant: des chélates de lanthanides (III) ou des nanoparticules en contenant; des nanoparticules polymères colorées; des matières fluorescentes ou des nanoparticules en contenant; des matières phosphorescentes ou des nanoparticules en contenant; des liposomes contenant des colorants; des enzymes; des matières super-paramagnétiques ou des nanoparticules en contenant; des nanoparticules métalliques; et des nanoparticules de carbone.

3. Dispositif microfluidique selon la revendication 1 ou 2, dans lequel le conjugué de marquage comprend un marqueur entraînant l'expression de signaux optiques de l'analyte de l'échantillon fluide, et le marqueur est combiné à l'analyte.

4. Dispositif microfluidique selon l'une quelconque des revendications 1 à 3, dans lequel le conjugué de marquage est au moins un conjugué parmi divers conjugués de marquage contenant des substances de marquage distinctes.

5. Dispositif microfluidique selon l'une quelconque des revendications 1 à 4, dans lequel le liant est choisi dans le groupe comprenant: anticorps, antigène, récepteur, ligand, oligonucléotide, haptène et aptamère.

6. Dispositif microfluidique selon l'une quelconque des revendications 1 à 5, dans lequel le liant de capture est lié à un site de réaction d'un analyte de l'échantillon fluide qui est différent d'un autre site de réaction auquel le conjugué de marquage réagit avec l'analyte.

7. Dispositif microfluidique selon l'une quelconque des revendications 1 à 6, dans lequel la seconde chambre de réaction comprend une zone de détection dans laquelle le liant de capture est fixé à ladite chambre de réaction.

8. Dispositif microfluidique selon l'une quelconque des revendications 1 à 7, comprenant en outre une unité de détection placée en dehors de la structure microfluidique, l'unité de détection comprenant:
une unité d'émission de lumière émettant de la lumière en direction de la chambre de détection de la structure microfluidique;
une unité de réception de lumière recevant la lumière émise par l'unité d'émission de lumière et ayant traversé la chambre de détection; et
une unité d'analyse analysant au moins une caractéristique optique de la lumière reçue par l'unité de réception de lumière et calculant la concentration de l'analyte de l'échantillon fluide.

9. Procédé d'immunoanalyse faisant appel à un dispositif microfluidique centrifuge selon l'une quelconque des revendications 1 à 8, le procédé d'immunoanalyse comprenant:
l'injection d'un échantillon fluide dans le dispositif microfluidique, la centrifugation de l'échantillon fluide afin de produire un surnageant, et le transfert du surnageant dans une première chambre de réaction du dispositif microfluidique;
la combinaison d'un analyte contenu dans le surnageant à un conjugué de marquage, comprenant un marqueur et un liant, contenu dans la première chambre de réaction afin de former un premier complexe immun, le liant se liant spécifiquement à l'analyte du surnageant;
la combinaison du premier complexe immun à un liant de capture choisi dans le groupe comprenant: anticorps, antigène, récepteur, ligand, oligonucléotide, haptène ou aptamère, contenu dans une seconde chambre de réaction afin de former un second complexe immun;
le transfert du conjugué de marquage n'ayant pas réagi, ainsi que des résidus réactionnels, vers une chambre de décharge conjointement à une solution de lavage provenant d'une chambre de lavage;
la dissociation du conjugué de marquage d'avec le second complexe immun dans la seconde chambre de réaction au moyen d'un tampon d'élution provenant d'une chambre de tampon du dispositif microfluidique;
le transfert du conjugué de marquage dissocié vers une chambre de détection du dispositif microfluidique; et
la mesure et l'analyse des caractéristiques optiques du marqueur du conjugué de marquage dissocié dans la chambre de détection.

10. Procédé d'immunoanalyse selon la revendication 9, dans lequel le conjugué de marquage comporte au moins un marqueur choisi dans le groupe comprenant: des chélates de lanthanides (III) ou des nanoparticules en contenant; des nanoparticules polymères colorées; des matières fluorescentes ou des nanoparticules en contenant; des matières phosphorescentes ou des nanoparticules en contenant; des liposomes contenant des colorants; des enzymes; des matières super-paramagnétiques ou des nanoparticules en contenant; des nanoparticules métalliques; et des nanoparticules de carbone.

11. Procédé d'immunoanalyse selon la revendication 9 ou 10, dans lequel le conjugué de marquage est au moins un conjugué de marquage parmi divers conjugués de marquage contenant des substances de marquage distinctes.

12. Procédé d'immunoanalyse selon la revendication 9, 10 ou 11, dans lequel le liant est choisi dans le groupe comprenant: anticorps, antigène, récepteur, ligand, oligonucléotide, haptène ou aptamère.

13. Procédé d'immunoanalyse selon l'une quelconque des revendications 9 à 12, dans lequel le liant de capture est lié à un site de réaction de l'analyte qui est différent d'un autre site de réaction auquel le conjugué de marquage réagit avec l'analyte.

14. Procédé d'immunoanalyse selon l'une quelconque des revendications 9 à 13, dans lequel la seconde chambre de réaction comprend une zone de détection dans laquelle le liant de capture est fixé à ladite chambre de réaction.

15. Procédé d'immunoanalyse selon l'une quelconque des revendications 9 à 14, comprenant en outre la détermination de la fluorescence du conjugué de marquage au moyen d'une mesure de la fluorescence à résolution temporelle qui mesure la fluorescence de la lumière reçue par une unité de réception de lumière de l'unité de détection pendant un temps résolu, la fluorescence de la lumière reçue par l'unité de réception de lumière étant mesurée après un décalage de temps prédéterminé.
